# EUROPEAN PATENT APPLICATION

(11) **EP 4 767 936 A1**
(43) Date of publication of application: **01.07.2026**
(21) Application number: 25188206.4
(22) Date of filing: 08.07.2025
(51) Int. Cl.: A61B 5/145, C08F 8/00, C08F 251/00

(54) **BIOSENSOR, OUTER MEMBRANE OF BIOSENSOR, METHODS OF PREPARATION AND USE THEREOF**

(30) Priority: 26.12.2024 CN 202411954334
(71) Applicant: Shanghai Microport Lifesciences Co., Ltd., Shanghai 201318 (CN)
(72) Inventor: ZHAO, Lili, Shanghai, 201318 (CN); CHEN, Xun, Shanghai, 201318 (CN); WILLIAMS, Nicholas Xavier, Shanghai, 201318 (CN); ZHOU, Pan, Shanghai, 201318 (CN)
(74) Representative: Marks & Clerk LLP

(57) **Abstract**

The present invention relates to a biosensor, an outer membrane of a biosensor, methods of preparation and use thereof. The outer membrane includes polymer molecules containing thioureido groups, which result from polymerization of a pyridyl-containing polymer with a thioureido-containing monomer. That is, thioureido groups in the monomer and pyridyl groups in the polymer are held together by chemical bonds. The thioureido groups in the outer membrane can absorb silver ions and block their escape from a reference electrode through osmosis, increasing biocompatibility of the outer membrane and reducing its toxicity.

## Description

### TECHNICAL FIELD

The present invention relates to the field of sensors and, in particular, to a biosensor, an outer membrane of a biosensor, methods of preparation and use thereof.

### BACKGROUND

Biosensors, as one of the technologies that have undergone rapid development and attracted extensive research effort in recent years, contain a bioactive material, such as enzyme, microorganism, animal or plant tissue, organelle, antigen, antibody, etc., and determine a concentration of an analyte based on an acoustic, optical, electrical or other signal converted by a chemical or physical transducer from information resulting from a biological reaction of the analyte with molecules that have recognized the analyte. Due to a wide range of advantages including high specificity, high sensitivity and fast response, biosensors are playing an increasingly important role in the field of medicine and providing a novel fast and simple approach for basic medical research and clinical diagnosis.

As devices for collecting physiological information of the human body, biosensors must be brought into contact with the human body, or even deployed within the human body. Therefore, they must possess good biocompatibility and high specificity to analytes of interest. Glucose sensors are established biosensors, which have been intensively studied and are available on the market. Most currently available glucose sensors can be effectively used for 14 days, and in order to extend this term of effective use, further improvement of their biocompatible layers is necessary. Structural analysis of such sensors, coupled with cytotoxicity assays, has revealed that escape of silver ions from a reference electrode through osmosis is an importance cause of their suboptimal biocompatibility because silver ions are somewhat biologically toxic when present in excess.

### SUMMARY

In view of the above, it would be desirable to provide a more biocompatible and less toxic biosensor, as well as an outer membrane and methods of preparation thereof.

Use of such a biosensor would also be desirable.

An outer membrane of a biosensor is provided herein, which includes polymer molecules containing thioureido groups, where the polymer molecules result from polymerization of a pyridyl-containing polymer with a thioureido-containing monomer.

In one embodiment, the pyridyl-containing polymer may be selected from one or more of poly(4-vinylpyridine-co-styrene), poly(4-vinylpyridine-co-butyl methacrylate) and poly(4-vinylpyridine), and the monomer may be selected from thioureas with one amino group and/or thioureas with two amino groups.

In one embodiment, the monomer may be selected from one or more of thiourea, 1-propylthiourea, 1-butylthiourea, N-benzylthiourea, anilinothiourea, phenylthiourea, cyclopentylthiourea, methylthiourea, guanylthiourea, ethylthiourea, isopropylthiourea, allylthiourea, aminothiourea and tert-butylthiourea.

In one embodiment, the thioureido-containing monomer may be at a molar ratio of 1: 100 to 30: 100 to pyridyl groups in the pyridyl-containing polymer.

Also provided herein is a method of preparing an outer membrane of a biosensor, which includes the steps of:
dissolving a pyridyl-containing polymer in an amide solvent, obtaining a polymer solution;
adding a bromo-terminated carboxylic acid to the polymer solution, which modifies the polymer so that the polymer contains carboxyl groups;
adding a thioureido-containing monomer to the modified polymer solution, causing a grafting reaction, purifying and drying a product of the grafting reaction, and dissolving the dried product in an aqueous ethanol solution, obtaining a grafted polymer solution; and
adding a crosslinking agent terminated with an epoxy ether with a functionality greater than two to the grafted polymer solution and dip-coating an implantable portion of the sensor in the solution, thereby forming the outer membrane of the biosensor.

In one embodiment, the pyridyl-containing polymer may be selected from one or more of poly(4-vinylpyridine-co-styrene), poly(4-vinylpyridine-co-butyl methacrylate) and poly(4-vinylpyridine), wherein the monomer is selected from thioureas with one amino group and/or thioureas with two amino groups; the bromo-terminated carboxylic acid is selected from one or more of 3-bromopropionic acid, 4-bromobutyric acid, 5-bromovaleric acid, 6-bromohexanoic acid, 8-bromooctanoic acid and 9-bromononanoic acid; and the crosslinking agent is selected from one or more of trimethylolpropane triglycidyl ether, pentaerythritol glycidyl ether, glycerol diglycidyl ether and poly(ethylene glycol) glycidyl ether.

In one embodiment, the amide solvent may be N,N-dimethylformamide or N,N-dimethylacetamide,
wherein the polymer is modified under conditions including a reaction temperature of 60-120 °C and a reaction duration of 12-72 hours.

In one embodiment, the grafting reaction may be run under any one or more of the following conditions:
(1) a reaction temperature of 20-50 °C; and
(2) a reaction duration of 12-72 hours.

In one embodiment, the coating may be accomplished by a process including: dip-coating in a solution having a solid content of 30-200 mg/ml 4-12 times, for 2-20 seconds each time; and curing following the dip-coating, which is conducted for 24-72 hours at a temperature of 37 °C and a humidity of 20-90%, wherein the solid content refers to presence of the crosslinking agent and the dried product in a total amount of 30-200 milligrams per milliliter of the solution.

Also provided herein is a method of preparing an outer membrane of a biosensor, which includes the steps of:
mixing a crosslinking agent terminated with an epoxy ether with a functionality greater than three with a thioureido-containing monomer, obtaining a mixed solution;
obtaining a polymer solution from dissolution of a pyridyl-containing polymer; and
adding the mixed solution to the polymer solution and performing dip-coating, thereby forming the outer membrane of the biosensor.

In one embodiment, the pyridyl-containing polymer may be selected from one or more of poly(4-vinylpyridine-co-styrene), poly(4-vinylpyridine-co-butyl methacrylate) and poly(4-vinylpyridine), wherein the monomer is selected from thioureas with one amino group and/or thioureas with two amino groups, and the crosslinking agent is selected from one or more of trimethylolpropane triglycidyl ether, pentaerythritol glycidyl ether, glycerol diglycidyl ether and poly(ethylene glycol) glycidyl ether.

In one embodiment, the step of obtaining the mixed solution by mixing the crosslinking agent with the thioureido-containing monomer may include:
dissolving the crosslinking agent and the thioureido-containing monomer in a first solvent and a second solvent, respectively, followed by mixing and stirring for 8-24 hours, thereby obtaining the mixed solution, wherein the first and second solvents are both ethanol solution each containing water and ethanol at a volume ratio of 1: 3.

In one embodiment, the coating may be accomplished by a process including: dip-coating in a solution having a solid content of 30-200 mg/ml 4-12 times, for 2-20 seconds each time; and curing following the dip-coating, which is conducted for 24-72 hours at a temperature of 37 °C and a humidity of 20-90%, wherein the solid content refers to presence of the crosslinking agent and a dried product in a total amount of 30-200 milligrams per milliliter of the solution.

Also provided herein is a biosensor including the outer membrane as defined above, or an outer membrane prepared according to any of the methods as defined above.

Also provided herein is use of the biosensor as defined above for detection of glucose, lactic acid, ketone bodies, alcohol or uric acid.

In the outer membrane as defined above, the polymer molecules containing thioureido groups are obtained as a result of polymerization of a pyridyl-containing polymer with a thioureido-containing monomer. That is, thioureido groups in the monomer and pyridyl groups in the polymer are held together by chemical bonds. The thioureido groups in the outer membrane can absorb silver ions and block their escape from a reference electrode through osmosis, increasing biocompatibility of the outer membrane and reducing its toxicity.

In methods as defined above, thioureido groups are incorporated into the outer membrane by crosslinking or grafting to absorb silver ions and block their escape from a reference electrode through osmosis, thereby increasing biocompatibility of the outer membrane. In addition, these methods are simple, provide ease of operation, and can be used to form various outer membranes of various biosensors. The resulting outer membranes can improve biocompatibility of the biosensors.

### BRIEF DESCRIPTION OF THE DRAWINGS

Fig. 1 shows a process flowchart of a method of preparing an outer membrane of a biosensor according to an embodiment.
Fig. 2 shows a process flowchart of a method of preparing an outer membrane of a biosensor according to another embodiment.

### DETAILED DESCRIPTION

In order to facilitate an understanding of the present invention, the invention is described more fully below with reference to the accompanying drawings, which show preferred embodiments for practicing the invention. However, the invention may be implemented in many different forms and should not be construed as being limited to the embodiments set forth herein. Rather, these embodiments are provided so that this disclosure will be thorough and complete.

Unless defined otherwise, all technical and scientific terms used herein have the same meaning as commonly understood by one of ordinary skill in the art to which this invention belongs. The terminology used herein is for the purpose of describing particular embodiments only and is not intended to be limiting of this invention.

As used herein, the phrase "one or more of ..." refers to any one, two or more of the listed items, and the term "several" means two or more.

As described herein, any percentage concentration refers to a final concentration, unless otherwise specified, which represents a proportion of a component in a system after it is added to the system.

As used herein, the words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

Where a numerical range is disclosed herein such range is continuous, and includes every value between the minimum and maximum values of such range. Further, where a range refers to integers, every integer between the minimum and maximum values of such range is included. In addition, where multiple ranges are provided to describe a feature or characteristic, such ranges can be combined. In other words, unless otherwise specified, all ranges disclosed herein are to be understood to encompass any and all sub-ranges subsumed therein.

Any embodiment described herein in an open-ended fashion as including one or more listed features encompasses both a closed-ended implementation consisting of the listed features and open-ended implementations each including the listed features.

As used herein, the terms "include", "have" and any variations thereof are intended to cover non-exclusive inclusions. For example, a process, method, system, article or apparatus that includes a list of steps or elements is not necessarily limited to the listed steps or elements, but may optionally further include other steps or elements not expressly listed or inherent to such process, method, article or apparatus.

Reference in this specification to "one embodiment" or "an embodiment" means that a particular feature, structure or characteristic described in connection with the embodiment is included in at least one embodiment of this application. Thus, the appearance of such phrases in various places in the specification is not necessarily all referring to the same embodiment, nor is a separate or alternative embodiment mutually exclusive of other embodiments. As would be explicitly and implicitly appreciated by those skilled in the art, embodiments described herein can be combined with other embodiments.

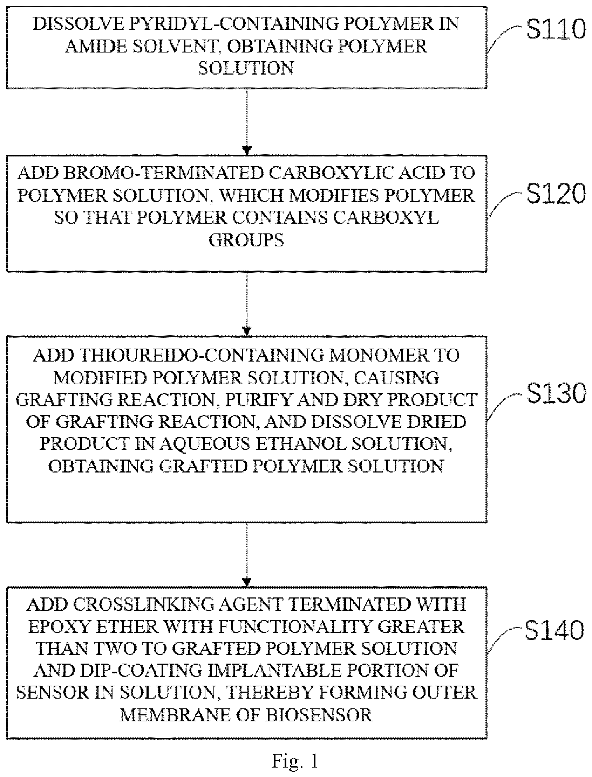

Referring to Fig. 1, a method of preparing an outer membrane of a biosensor according to an embodiment includes the step as follows.

Step S110: Dissolve a pyridyl-containing polymer in an amide solvent, obtaining a polymer solution.

The pyridyl-containing polymer may be selected from one or more of poly(4-vinylpyridine-co-styrene), poly(4-vinylpyridine-co-butyl methacrylate) and poly(4-vinylpyridine). The amide solvent may be N,N-dimethylformamide and/or N,N-dimethylacetamide. Pyridyl groups are introduced to facilitate subsequent introduction of thioureido groups. In the present embodiment, the pyridyl-containing polymer is poly(4-vinylpyridine-co-styrene), and the dimethylamide is N,N-dimethylformamide. The dissolution of poly(4-vinylpyridine-co-styrene) in the dimethylamide may occur at ambient temperature.

Step S120: Add a bromo-terminated carboxylic acid to the polymer solution, which modifies the polymer by carboxyl groups thereto.

The bromo-terminated carboxylic acid may be selected from one or more of 3-bromopropionic acid, 4-bromobutyric acid, 5-bromovaleric acid, 6-bromohexanoic acid, 8-bromooctanoic acid and 9-bromononanoic acid. In the present embodiment, the bromo-terminated carboxylic acid is 4-bromobutyric acid. The polymer may be modified under conditions including a reaction temperature of 60-120 °C and a reaction duration of 12-72 hours. Preferably, the reaction temperature may be 80-100 °C, and the reaction duration may be 20-36 hours. A molar ratio of the bromo-terminated carboxylic acid to the subsequently introduced thiourea may be 1: 1.

In particular, after a pyridyl-containing polymer is completely dissolved in an amide solvent in step S110, a bromo-terminated carboxylic acid is added to the resulting polymer solution, inducing a nucleophilic substitution reaction. The reaction may be run at 60-120 °C for 12-72 hours to graft the bromo-terminated carboxylic acid onto the pyridyl-containing polymer so that the polymer contains carboxyl groups. In this process, nitrogen atoms in the pyridyl groups and bromine atoms in the bromo-terminated carboxylic acid are ionized into nitrogen cations and bromide anions, respectively, which are held together by ionic bonds, thus grafting the bromo-terminated carboxylic acid to the pyridyl-containing polymer.

Step S130: Add a thioureido-containing monomer to the modified polymer solution, causing a grafting reaction. A product of the grafting reaction is purified and dried, and the dried product is dissolved in an aqueous ethanol solution, obtaining a polymer solution.

In particular, after the modification reaction in step S120, a thioureido-containing monomer may be added to the modified polymer solution, causing a grafting reaction. The reaction may be run at 20-50 °C for 12-72 hours, allowing amino groups in the monomer to be condensed with the carboxyl groups in the modified polymer, thus grafting the thioureido-containing monomer onto side chains of the modified polymer. A product of the grafting reaction may be then purified and dried into a powder polymer, which may be dissolved in an aqueous ethanol solution, obtaining a polymer solution. Preferably, the aqueous ethanol solution contains water and ethanol at a volume ratio of 1: 3.

In one embodiment, the thioureido-containing monomer may be selected from thioureas with one amino group and/or thioureas with two amino groups. Preferably, the thioureido-containing monomer may be selected from one or more of thiourea, 1-propylthiourea, 1-butylthiourea, N-benzylthiourea, anilinothiourea, phenylthiourea, cyclopentylthiourea, methylthiourea, guanylthiourea, ethylthiourea, isopropylthiourea, allylthiourea, aminothiourea and tert-butylthiourea.

In one embodiment, the thioureido-containing monomer may be added at a molar ratio of 1: 100 to 30: 100, preferably 20: 100, to the pyridyl groups in the pyridyl-containing polymer.

Step S140: Add a crosslinking agent terminated with an epoxy ether with a functionality greater than two to the purified solution and dip-coat an implantable portion of the sensor in the solution, thereby forming the outer membrane.

In particular, a crosslinking agent terminated with an epoxy ether with a functionality greater than two may be added to the purified solution from step S130, obtaining a coating solution. An implantable portion of the sensor may be then dip-coated in the coating solution, thus forming the outer membrane on the exterior of the implantable portion. In this way, the thioureido groups are present at the exterior of the sensor. The crosslinking agent may be selected from one or more of trimethylolpropane triglycidyl ether, pentaerythritol glycidyl ether, glycerol diglycidyl ether and poly(ethylene glycol) glycidyl ether.

In one embodiment, the dip-coating process may involve 4-12 dip-coating cycles, each lasting for 2-20 seconds, using a solution having a solid content of 30-200 mg/ml and be followed by curing at a temperature of 37 °C and a humidity of 20%-90% for 24-72 hours. Here, the solid content refers to presence of the crosslinking agent and the dried product in a total amount of 30-200 milligrams per milliliter of the solution.

The above method has at least the following advantages:
(1) Through grafting the thioureido-containing monomer onto side chains of the pyridyl-containing polymer, the obtained outer membrane contains thioureido groups, which can absorb silver ions and block their escape from an electrode through osmosis, increasing biocompatibility of the outer membrane.

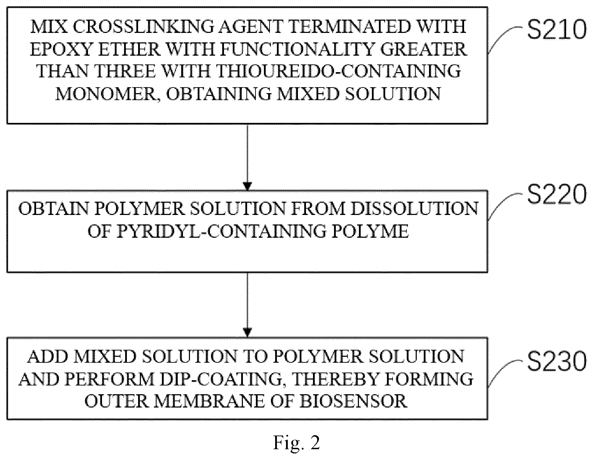

Referring to Fig. 2, a method of preparing an outer membrane of a biosensor according to another embodiment includes the step as follows.

Step S210: Mix a crosslinking agent terminated with an epoxy ether with a functionality greater than three and a thioureido-containing monomer, obtaining a mixed solution.

Optionally, a crosslinking agent terminated with an epoxy ether with a functionality greater than three and a thioureido-containing monomer may be dissolved in a first solvent and a second solvent, respectively, and the resulting solutions may be mixed and stirred for 8-24 hours, obtaining a mixed solution. The first and second solvents may be both ethanol solutions each containing water and ethanol at a volume ratio of 1: 3. During the mixing of the crosslinking agent with the thioureido-containing monomer, a ring-opening addition reaction may occur between amino groups in the monomer and epoxy groups in the crosslinking agent.

Notably, either or both of the first and second solvents may also be ethyl acetate or the like, and the water/ethanol volume ratio in the ethanol solutions may also be 1: 3, 1: 5, etc. The crosslinking agent may be selected from one or more of trimethylolpropane triglycidyl ether, pentaerythritol glycidyl ether and glycerol diglycidyl ether. The thioureido-containing monomer may be selected from thioureas with one amino group and/or thioureas with two amino groups. Preferably, the thioureido-containing monomer may be selected from one or more of thiourea, 1-propylthiourea, 1-butylthiourea, N-benzylthiourea, anilinothiourea, phenylthiourea, cyclopentylthiourea, methylthiourea, guanylthiourea, ethylthiourea, isopropylthiourea, allylthiourea, aminothiourea and tert-butylthiourea.

S220: Dissolve a pyridyl-containing polymer, obtaining a polymer solution.

The pyridyl-containing polymer may be selected from one or more of poly(4-vinylpyridine-co-styrene), poly(4-vinylpyridine-co-butyl methacrylate) and poly(4-vinylpyridine). It may be dissolved in dimethylamide as a solvent. The dimethylamide may be N,N-dimethylformamide and/or N,N-dimethylacetamide. The pyridyl groups are introduced to facilitate subsequent introduction of thioureido groups. In the present embodiment, the pyridyl-containing polymer is poly(4-vinylpyridine-co-styrene), and the dimethylamide is N,N-dimethylformamide. The dissolution of poly(4-vinylpyridine-co-styrene) in the dimethylamide may occur at ambient temperature.

In one embodiment, the thioureido-containing monomer may be present at a molar ratio of 1: 100 to 30: 100, preferably 20: 100, to the pyridyl groups in the pyridyl-containing polymer.

S230: Add the mixed solution to the polymer solution and carry out a dip-coating process, thereby forming the outer membrane.

In particular, the mixed solution from step S210 may be added to the polymer solution from step S220, forming a new mixed solution. An implantable portion of the sensor may be dip-coated in the new mixed solution, thereby forming the outer membrane on the exterior of the implantable portion. In this way, the thioureido groups are present at the exterior of the sensor.

The above method has at least the following advantages:
The thioureido groups, which are introduced into the outer membrane by crosslinking, can absorb silver ions and block them from escaping from an electrode through osmosis, increasing biocompatibility of the outer membrane. In addition, the use of the crosslinking approach enables simple preparation by easy operation.

In an embodiment of the present invention, there is provided an outer membrane of a biosensor, which contains polymer molecules having thioureido groups. The polymer molecules are obtained as a result of polymerization of a pyridyl-containing polymer with a thioureido-containing monomer.

The pyridyl-containing polymer may be selected from one or more of poly(4-vinylpyridine-co-styrene), poly(4-vinylpyridine-co-butyl methacrylate) and poly(4-vinylpyridine). The thioureido-containing monomer may be selected from thioureas with one amino group and/or thioureas with two amino groups. Preferably, the thioureido-containing monomer may be selected from one or more of thiourea, 1-propylthiourea, 1-butylthiourea, N-benzylthiourea, anilinothiourea, phenylthiourea, cyclopentylthiourea, methylthiourea, guanylthiourea, ethylthiourea, isopropylthiourea, allylthiourea, aminothiourea and tert-butylthiourea. The thioureido-containing monomer may be present at a molar ratio of 1: 100 to 30: 100, preferably 20: 100, to pyridyl groups in the pyridyl-containing polymer.

In particular, the outer membrane may be prepared according to any of the methods described above in connection with the foregoing embodiments. Detailed description of the methods is not repeated here for the sake of brevity.

The outer membrane contains polymer molecules having thioureido groups, which are obtained as a result of polymerization of a pyridyl-containing polymer with a thioureido-containing monomer. That is, thioureido groups in the monomer and pyridyl groups in the polymer are held together by chemical bonds. The thioureido groups in the outer membrane can absorb silver ions and block their escape from a reference electrode through osmosis, increasing biocompatibility of the outer membrane and reducing its toxicity.

In an embodiment of the present invention, there is provided a biosensor including the outer membrane as defined above, or an outer membrane obtainable according to any of the methods described above in connection with the foregoing embodiments. This biosensor provides good biocompatibility.

In an embodiment of the present invention, there is also provided use of the biosensor for detection of glucose, lactic acid, ketone bodies, alcohol or uric acid.

The biosensor has good biocompatibility and various biological enzymes can be incorporated therein to detect a substance such as glucose, lactic acid, a ketone body, alcohol, uric acid, or the like.

The biosensor of the present invention and benefits thereof will be described in greater detail below by way of specific examples, and objects and advantages of the invention will become apparent from the following detailed description. It should be understood that the specific examples described herein are solely for the purpose of illustration and not provided as a limitation of the invention. Unless otherwise particularly specified, the following examples do not include other components than those described herein, except for inevitable impurities. The reagents and instruments used in the examples are selected as conventional ones available in the art, unless otherwise particularly specified. Any experimental method in the examples, if specific conditions are not given, is carried out under conventional conditions, for example, as described in literature and books, or as recommended by manufacturers.

### Example 1

An outer membrane of a biosensor was prepared in this example according to a method as described below.
(1) 10 g of poly(4-vinylpyridine-co-styrene) was dissolved in 100 ml of N,N-dimethylformamide at ambient temperature.
(2) 0.28 g of 4-bromobutyric acid was added to the polymer solution resulting from step (1), causing a reaction. The reaction was run at 110 °C for 48 hours.
(3) 0.15 g of methylthiourea was added to the modified polymer solution from step (2), causing a grafting reaction. The reaction was run at 45 °C for 24 hours. The product of the grafting reaction was obtained as a polymer, which was then precipitated in an ether solution. After small molecule impurities were removed, the product was dried in a vacuum oven at 50 °C for 24 hours. Finally, the dried product was dissolved in an aqueous ethanol solution (containing water and ethanol at a volume ratio of 1: 3), obtaining a polymer solution with a solid content of 100 mg/ml. The thioureido-containing monomer was added at a molar ratio of 1: 100 to pyridyl groups in the pyridyl-containing polymer.
(4) Poly(ethylene glycol) glycidyl ether was added, as a crosslinking agent, to an aqueous ethanol solution (containing ethanol and water at a volume ratio of 3: 1), obtaining a solution having a solid content of 100 mg/ml. The resulting solution was then added to the polymer solution from step (3), obtaining a dip-coating solution. An implantable portion of the sensor was dipped in the dip-coating solution 6 times, for 4 seconds each time. After the dip-coating process was completed, curing was carried out at a temperature of 37 °C and a humidity of 50% for 24 hours.

### Example 2

An outer membrane of a biosensor was prepared in this example according to a method similar to that of Example 1, except that the thioureido-containing monomer was added at a molar ratio of 5: 100 to pyridyl groups in the pyridyl-containing polymer.

### Example 3

An outer membrane of a biosensor was prepared in this example according to a method similar to that of Example 1, except that the thioureido-containing monomer was added at a molar ratio of 10: 100 to pyridyl groups in the pyridyl-containing polymer.

### Example 4

An outer membrane of a biosensor was prepared in this example according to a method similar to that of Example 1, except that the thioureido-containing monomer was added at a molar ratio of 20: 100 to pyridyl groups in the pyridyl-containing polymer.

### Example 5

An outer membrane of a biosensor was prepared in this example according to a method similar to that of Example 1, except that the thioureido-containing monomer was added at a molar ratio of 30: 100 to pyridyl groups in the pyridyl-containing polymer.

### Comparative Example 1

A biosensor was prepared in this example according to a method as described below.
(1) 10 g of poly(4-vinylpyridine-co-styrene) was dissolved in 100 ml of an aqueous ethanol solution (containing ethanol and water at a volume ratio of 3: 1) at ambient temperature.
(2) Poly(ethylene glycol) glycidyl ether was added, as a crosslinking agent, to an aqueous ethanol solution (containing ethanol and water at a volume ratio of 3: 1), obtaining a solution having a solid content of 100 mg/ml. The resulting solution was then added to the solution from step (1), obtaining a dip-coating solution. An implantable portion of the sensor was dipped in the dip-coating solution 6 times, for 4 seconds each time. After the dip-coating process was completed, curing was carried out at a temperature of 37 °C and a humidity of 50% for 24 hours.

### Example 6

(1) 0.76 g of trimethylolpropane triglycidyl ether and 0.1 g of thiourea were separately weighed and dissolved in separate aqueous ethanol solutions (each containing water and ethanol at a volume ratio of 1: 3). The resulting two solutions were mixed and stirred for 8 hours, obtaining a mixed solution with a solid content of 100 mg/ml.
(2) Poly(4-vinylpyridine-co-styrene) was dissolved in an aqueous ethanol solution (containing water and ethanol at a volume ratio of 1: 3) at ambient temperature, obtaining a poly(4-vinylpyridine-co-styrene) solution having a solid content of 100 mg/ml.
(3) The mixed solution from step (1) was added to the polymer solution from step (2), obtaining a dip-coating solution. An implantable portion of the sensor was dipped in the dip-coating solution 6 times, for 4 seconds each time. After the dip-coating process was completed, curing was carried out at a temperature of 37 °C and a humidity of 50% for 24 hours. Finally, the thioureido-containing monomer was present at a molar ratio of 5: 100 to pyridyl groups in the pyridyl-containing polymer.

### Example 7

An outer membrane of a biosensor was prepared in this example according to a method similar to that of Example 5, except that the thioureido-containing monomer was present at a molar ratio of 10: 100 to pyridyl groups in the pyridyl-containing polymer and that the dip-coating process involved 4 dip-coating cycles.

### Example 8

An outer membrane of a biosensor was prepared in this example according to a method similar to that of Example 5, except that the thioureido-containing monomer was present at a molar ratio of 10: 100 to pyridyl groups in the pyridyl-containing polymer.

### Example 9

An outer membrane of a biosensor was prepared in this example according to a method similar to that of Example 5, except that the thioureido-containing monomer was present at a molar ratio of 20: 100 to pyridyl groups in the pyridyl-containing polymer.

### Comparative Example 2

(1) 1 g of trimethylolpropane triglycidyl ether was dissolved, as a crosslinking agent, to an aqueous ethanol solution (containing water and ethanol at a volume ratio of 1: 3), obtaining a crosslinking agent solution having a solid content of 100 mg/ml.
(2) Poly(4-vinylpyridine-co-styrene) was dissolved in an aqueous ethanol solution (containing water and ethanol at a volume ratio of 1: 3) at ambient temperature, obtaining a poly(4-vinylpyridine-co-styrene) solution having a solid content of 100 mg/ml.
(3) The solution from step (1) was added to the polymer solution from step (2), obtaining a dip-coating solution. An implantable portion of the sensor was dipped in the dip-coating solution 6 times, for 4 seconds each time. After the dip-coating process was completed, curing was carried out at a temperature of 37 °C and a humidity of 50% for 24 hours.

Outer membranes prepared according to the above examples were subjected to the following assays.

### 1. Silver Ion Concentration Test

The prepared sensors were soaked in phosphate buffered saline (PBS) (10 samples in 10 ml of PBS) for 14 days (shaken at 50 rpm/min in an air bath at a constant temperature of 37 °C). After that, the sensors were taken out, and the soaking solutions were subjected to a silver ion concentration test using an inductively coupled plasma mass spectrometry (ICP-MS) instrument. A calibration line was plotted using standard silver ion solutions at the beginning of the test, and an equal amount was measured for each solution.

### 2. In Vitro Cytotoxicity Test

An in vitro cytotoxicity test was performed using the MTT assay.

The silver ion concentration test was carried out on the implantable portions of the sensors prepared according to Examples 1 to 9 and Comparative Examples 1 to 2, and the in vitro cytotoxicity test was performed on Examples 4 and 8 and Comparative Examples 1 to 2. The results are shown in Tables 1 and 2 below.

**Table 1**

| Example | Thioureido-to-Pyridyl Molar Ratio | Silver Ions (ppb) | Amount of Absorption | Rate of Absorption | In Vitro Cytotoxicity |
|---|---|---|---|---|---|
| Example 1 | 1: 100 | 4.618 | 5.976 | 56.41% | / |
| Example 2 | 5: 100 | 1.496 | 9.098 | 85.88% | / |
| Example 3 | 10: 100 | 0.503 | 10.091 | 95.25% | / |
| Example 4 | 20: 100 | 0.092 | 10.502 | 99.13% | 74.7% |
| Example 5 | 30: 100 | 0.035 | 10.502 | 99.67% | / |
| Comparative Example 1 | / | 10.594 | | | 37.8% |

**Table 2**

| Example | Thioureido-to-Pyridyl Molar Ratio | Silver Ions (ppb) | Amount of Absorption | Rate of Absorption | In Vitro Cytotoxicity |
|---|---|---|---|---|---|
| Example 6 | 5: 100 | 1.038 | 5.841 | 84.91% | / |
| Example 7 | 10: 100 | 0.713 | 6.166 | 89.64% | / |
| Example 8 | 10: 100 | 0.485 | 6.394 | 92.95% | / |
| Example 9 | 20: 100 | 0.045 | 6.834 | 99.35% | 78.5% |
| Comparative Example 2 | | 6.879 | | | 42.3% |

As can be seen from Tables 1 and 2, introducing more thioureido groups through grafting or crosslinking enables absorption of more silver ions that escape from an electrode through osmosis and can better block them from escape. The silver ion concentration test results of Comparative Examples 1 and 2 reveal that the absence of thioureido groups leads to higher concentrations, indicating poor absorption of silver ions. Therefore, this also suggests that the introduction of thioureido groups can enhance absorption of silver ions. In addition, the results of the in vitro cytotoxicity test on Examples 4 and 9 and Comparative Examples 1 and 2 show lower in vitro cytotoxicity of Examples 4 and 9, indicating that the introduction of thioureido groups can also improve sensor biocompatibility.

The values "0", "2.2", "11", "18" and "25" in the first column of Table 3 represent glucose concentrations, and the data in the columns "Example 1" to "Example 9" and "Comparative Example 1" to "Comparative Example 2" presents currents measured at the glucose concentrations. R represents a linear correlation coefficient, and the "Response" values are measured in nA/mmol. As can be seen from the response and linear correlation coefficient (R) measurements of Examples 1 to 5 given in Table 3, as the thiourea content increases, the response R initially rises, but starts to decline when reaching a certain value. Lower R

**Table 3**

| / | Example 1 | Example 2 | Example 3 | Example 4 | Example 5 | Example 6 | Example 7 | Exampl e 8 | Example 9 | Comparativ e Example 1 | Comparative Example 2 |
|---|---|---|---|---|---|---|---|---|---|---|---|
| 0 | 0.33 | 0.4 | 0.19 | 0.54 | 0.49 | 0.41 | 0.68 | 0.44 | 0.45 | 0.25 | 0.36 |
| 2.2 | 1.76 | 2.4 | 2.14 | 2.3 | 4.58 | 2.18 | 2.84 | 2.62 | 2.67 | 1.34 | 1.83 |
| 11 | 6.17 | 8.1 | 9.76 | 11.59 | 16.52 | 8.67 | 10.83 | 10.18 | 12.16 | 4.23 | 5.45 |
| 18 | 9.31 | 12.53 | 15.34 | 18.23 | 23.03 | 13.45 | 16.71 | 16.03 | 19.08 | 6.84 | 8.35 |
| 25 | 12.54 | 17.29 | 20.25 | 24.64 | 27.43 | 17.83 | 21.93 | 21.56 | 25.91 | 10.12 | 11.63 |
| Response | 0.48 | 0.66 | 0.81 | 0.98 | 1.09 | 0.70 | 0.85 | 0.84 | 1.02 | 0.38 | 0.44 |
| R | 0.9992 | 0.9996 | 0.9991 | 0.9996 | 0.9870 | 0.9993 | 0.9991 | 0.9997 | 0.9998 | 0.9977 | 0.9991 |

values are obtained when the thiourea content exceeds 20. This is because the resulting polymer becomes more and more hydrophilic and increasingly poorer in film-forming properties, as the thiourea content increases from a certain value. Accordingly, the inferior film-forming properties and lower sensor linearity of Example 5 (see the R values of Examples 4 and 5) are attributable to the high grafting rate of thiourea, which is as high as 30%. Lower sensor linearity means poorer sensor performance. The results of Examples 6 to 9 also suggest that sensor linearity starts to degrade when the thiourea content reaches a certain value.

The various technical features of the foregoing embodiments may be combined in any way. Although not all such combinations have been described above for the sake of brevity, any of them is considered to fall within the scope of this specification as long as there is no contradiction between the technical features.

Presented above are merely several embodiments of the present invention. Although these embodiments are described with some particularity and in some detail, it should not be construed that they limit the scope of the present application in any sense. It is to be noted that various variations and modifications can be made by those of ordinary skill in the art without departing from the concept of the present application. Accordingly, it is intended that all such variations and modifications are embraced within the scope of this application as defined in the appended claims. It should be understood that embodiments obtained by those skilled in the art from logical analysis, reasoning or limited experimentation in light of the teachings herein are all within the scope of the invention as defined in the appended claims. Thus, the true scope of the invention is defined by the appended claims, and the description and the accompanying drawings are presented for the purpose of explaining the claims.

## Claims

1. An outer membrane of a biosensor, comprising polymer molecules containing thioureido groups, which result from polymerization of a pyridyl-containing polymer with a thioureido-containing monomer.

2. The outer membrane according to claim 1, wherein the pyridyl-containing polymer is selected from one or more of poly(4-vinylpyridine-co-styrene), poly(4-vinylpyridine-co-butyl methacrylate) and poly(4-vinylpyridine), and the monomer is selected from thioureas with one amino group and/or thioureas with two amino groups.

3. The outer membrane according to claim 1, wherein the monomer is selected from one or more of thiourea, 1-propylthiourea, 1-butylthiourea, N-benzylthiourea, anilinothiourea, phenylthiourea, cyclopentylthiourea, methylthiourea, guanylthiourea, ethylthiourea, isopropylthiourea, allylthiourea, aminothiourea and tert-butylthiourea.

4. The outer membrane according to claim 1, wherein the thioureido-containing monomer is at a molar ratio of 1: 100 to 30: 100 to pyridyl groups in the pyridyl-containing polymer.

5. A method of preparing an outer membrane of a biosensor, comprising the steps of:
dissolving a pyridyl-containing polymer in an amide solvent, obtaining a polymer solution;
adding a bromo-terminated carboxylic acid to the polymer solution, which modifies the polymer so that the polymer contains carboxyl groups;
adding a thioureido-containing monomer to the modified polymer solution, causing a grafting reaction, purifying and drying a product of the grafting reaction, and dissolving the dried product in an aqueous ethanol solution, obtaining a grafted polymer solution; and
adding a crosslinking agent terminated with an epoxy ether with a functionality greater than two to the grafted polymer solution and dip-coating an implantable portion of the sensor in the solution, thereby forming the outer membrane of the biosensor.

6. The method of claim 5, wherein the pyridyl-containing polymer is selected from one or more of poly(4-vinylpyridine-co-styrene), poly(4-vinylpyridine-co-butyl methacrylate) and poly(4-vinylpyridine); the monomer is selected from thioureas with one amino group and/or thioureas with two amino groups; the bromo-terminated carboxylic acid is selected from one or more of 3-bromopropionic acid, 4-bromobutyric acid, 5-bromovaleric acid, 6-bromohexanoic acid, 8-bromooctanoic acid and 9-bromononanoic acid; and the crosslinking agent is selected from one or more of trimethylolpropane triglycidyl ether, pentaerythritol glycidyl ether, glycerol diglycidyl ether and poly(ethylene glycol) glycidyl ether.

7. The method of claim 5, wherein the amide solvent is N,N-dimethylformamide or N,N-dimethylacetamide, and
the polymer is modified under conditions including a reaction temperature of 60-120 °C and a reaction duration of 12-72 hours.

8. The method of claim 5, wherein the grafting reaction is run under any one or more of the following conditions:
(1)a reaction temperature of 20-50 °C; and
(2)a reaction duration of 12-72 hours.

9. The method of claim 5, wherein the coating is accomplished by a process comprising: dip-coating in a solution having a solid content of 30-200 mg/ml 4-12 times, for 2-20 seconds each time; and curing following the dip-coating, which is conducted for 24-72 hours at a temperature of 37 °C and a humidity of 20-90%, wherein the solid content refers to presence of the crosslinking agent and the dried product in a total amount of 30-200 milligrams per milliliter of the solution.

10. A method of preparing an outer membrane of a biosensor, comprising the steps of:
mixing a crosslinking agent terminated with an epoxy ether with a functionality greater than three with a thioureido-containing monomer, obtaining a mixed solution;
obtaining a polymer solution from dissolution of a pyridyl-containing polymer; and
adding the mixed solution to the polymer solution and performing dip-coating, thereby forming the outer membrane of the biosensor.

11. The method of claim 10, wherein the pyridyl-containing polymer is selected from one or more of poly(4-vinylpyridine-co-styrene), poly(4-vinylpyridine-co-butyl methacrylate) and poly(4-vinylpyridine); the monomer is selected from thioureas with one amino group and/or thioureas with two amino groups; and the crosslinking agent is selected from one or more of trimethylolpropane triglycidyl ether, pentaerythritol glycidyl ether, glycerol diglycidyl ether and poly(ethylene glycol) glycidyl ether.

12. The method of claim 10, wherein the step of obtaining the mixed solution by mixing the crosslinking agent with the thioureido-containing monomer comprises:
dissolving the crosslinking agent and the thioureido-containing monomer in a first solvent and a second solvent, respectively, followed by mixing and stirring for 8-24 hours, thereby obtaining the mixed solution, wherein the first solvent and the second solvent are both ethanol solution each containing water and ethanol at a volume ratio of 1: 3.

13. The method of claim 9, wherein the coating is accomplished by a process comprising: dip-coating in a solution having a solid content of 30-200 mg/ml 4-12 times, for 2-20 seconds each time; and curing following the dip-coating, which is conducted for 24-72 hours at a temperature of 37 °C and a humidity of 20-90%, wherein the solid content refers to presence of the crosslinking agent and a dried product in a total amount of 30-200 milligrams per milliliter of the solution.

14. A biosensor, comprising the outer membrane of any one of claims 1 to 4, or an outer membrane prepared according to the method of any one of claims 5 to 9, or an outer membrane prepared according to the method of any one of claims 10 to 13.

15. Use of the biosensor of claim 14 for detection of glucose, lactic acid, ketone bodies, alcohol or uric acid.
